# EUROPEAN PATENT SPECIFICATION

(11) **EP 1 064 900 B1**
(45) Date of publication and mention of the grant of the patent: **12.04.2006**
(21) Application number: 99830424.0
(22) Date of filing: 02.07.1999
(51) Int. Cl.: A61F 13/15, D04H 13/00, B32B 27/12

(54) **Sheet material comprising a plastic film and a web of fibers, method and plant for its production and its uses**
Bahnmaterial mit einem Kunststoffilm und einer Faserbahn, Verfahren und Vorrichtung zu dessen Herstellung sowie dessen Verwendungen
Matériau en feuille comprenant une feuille en matière plastique et une bande fibreuse, procédé et dispositif pour sa production et ses utilisations

(43) Date of publication of application: 03.01.2001
(73) Proprietor: Fintex and Partners Italia S.p.A., 51100 Pistoia (IT)
(72) Inventor: Giacometti, Claudio, 51030 Pieve a Celle, Pistoia (IT)
(74) Representative: Mannucci, Michele

(56) References cited:
- DE-A- 4 311 867
- US-A- 4 178 407
- US-A- 5 066 348
- US-A- 5 543 206
- US-A- 5 599 420
- US-A- 5 843 057

## Description

### Technical field

The present invention concerns a product consisting of a plastic film and a web of textile fibers, and its uses.

The invention also concerns a process for producing a sheet product consisting of a laminate of at least one film of plastic material combined with a web of textile fibers.

### State of the Art

In the production of many hygienic products, such as for example sanitary towels, incontinence pads, diapers and similar products, there is a frequent need for an impermeable or partially impermeable sheet product which is fluffy, soft and hypoallergenic. Moreover the product, while having the impermeable and/or resistant characteristics typical of a plastic film, must also have the feel of a woven fabric. This is because the sheet product will be in contact with the skin of the user of the absorbent product and the user will perceive contact with a plastic film as something unpleasant, particularly because it tends to adhere to the skin.

In producing sanitary towels or diapers the back sheet is ever more frequently made of a sheet material with a woven consistency on the outside. This is in order to give the user an increased feeling of comfort. Various processes have therefore been studied for obtaining a combined product consisting of a plastic film and a layer of textile fibers, in US-A-2,897,109 a method is described in which a thermoplastic film is extruded directly onto a web of consolidated textile fibers as it is unrolled. The two layers are combined by means of lamination thanks to the viscous consistency of the freshly extruded plastic film. Therefore the fibers of the non-woven web are anchored to the film and partially enclosed. The resulting product is particularly rigid and is not soft due to the partial endosure of the fibers in the film, and due to the fact that the fibers must be in the form of non-woven fibers consolidated before combining with the film.

US-A-2,896,626 describes a plastic film combined with a web of cellulose fibers, anchored to a plastic film for partial enclosure within it. A similar solution is described in FR-A-2,455,884. In both cases there is the disadvantage that the plastic film can become permeable to liquids due to the process of softening and partial melting necessary for the enclosure of the fibers. Moreover the partial melting of the film and the enclosure of the fibers within the film gives a relatively inflexible and not sufficiently soft product.

In US-A-4,725,473 a process is described for obtaining a product comprising a plastic film and a web of un-consolidated textile fibers. According to this known method, the combining is obtained exclusively by spot bonding, laminating the film and the web of un-consolidated textile fibers between two cylinders, one of which is provided with protuberances. To obtain a sufficiently reliable combining it is necessary to create a large bonding area, with consequent hardening of the material. In spite of the considerable extension of the bonding area (which according to this patent may reach 50% of the entire contact surface between the web and the film) the fibers in the intermediate area between the bonding points are completely free, except for the mechanical adhesion between fibers, and they are therefore subject to detachment. Moreover, the minimum bonded surface is equal to 5%, since below this value the fibers detach from the film completely. If the bonding area is increased above 5% with respect to the total area the product is excessively rigid.

From EP-A-0 691 203 a process is known for combining by hot lamination a web of textile fibers at least partially consolidated and a plastic film. The product obtained is not sufficiently soft and fluffy due to the extension of the area of bonding and the use of a web of partially consolidated fibers.

In EP-A-0 738 505 a process is described for the combining of a web of un-consolidated fibers to a plastic film in which the union between the two components occurs due to the softening and partial melting of the fibers, mainly without melting of the film. In this manner a more reliably impermeable product is produced, but it can be improved as far as the softness, the fluffiness of the layer of fibers and the overall flexibility of the sheet product are concerned.

A process according to the preamble of claim 1 is known from US-A-5,843,057.

### Aims and summary of the invention

The aim of the present invention is to realise a process for manufacturing a composite sheet product comprising at least one plastic film and at least one web of fibers combined together, which has superior characteristics of softness, flexibility and reliable impermeability compared to the products presently known.

These and further aims and advantages, which will become clear to experts in the field on reading the text which follows, are obtained with a process according to claim 1.

While, In theory it is possible to apply the adhesive either to the web of fibers or to the film, according to the preferred embodiment of the method of the present invention the adhesive is applied to the film. In this manner it remains on the surface of the film and is not dispersed within the thickness of the web of fibers.

During lamination it is possible to obtain at least partial consolidation of the fibers by means of adhesive and/or by partial melting of the fibers.

In essence the Invention foresees that a thermoplastic film - which can be a film of Polythene, possibly consisting of layers of different composition - is applied to a web of un-consolidated fibers with considerable fluffiness and softness with a process which is at least in part a gluing process, without melting the fibers and/or the web, or (as the following will make clear) using limited melting in discrete areas.

The term web of un-consolidated (or non-cohering) fibers means a sheet complex consisting of fibers united mainly by mechanical effects, that is by friction between fibers and by the form of the fibers which weave together.

A web of this type can be obtained, for example, by a carding process or by a flow of air which generates a web on a suction cylinder. Alternatively it is possible to use a humid web production process, with techniques similar to those for the production of paper. The 'spun-bonded' technique for the production of the web is not to be excluded.

The web of un-consolidated fibers is characterized by considerable softness and fluffiness with low consistency and mechanical resistance. The combining of the plastic film by adhesive, while anchoring the web of fibers to the film leaves the fibers sufficiently voluminous and free creating considerable fluffiness.

Since the web of un-consolidated textile fibers has low mechanical resistance, it is preferably produced in the same line on which the combining with the plastic film takes place. A single plant can be foreseen for this purpose including a system for the unrolling of the film from a bobbin, or an in-line extrusion system for the film, and a production machine for the web of un-consolidated textile fibers, which will generate a web which is laid on the film immediately after the adhesive has been applied, lamination of the two components will take place after application of the adhesive.

The adhesive may be of any type: pressure, emulsion (for example acrylic) or heat-sensitive. According to the type of adhesive used the material is cold or hot laminated. In a currently preferred embodiment the adhesive is of the hot-melt i.e. heat-sensitive type. It is activated by bringing it to a suitable temperature. This temperature is normally low with respect to the temperature at which the fibers and the plastic film soften, for example around 50-110°C and preferably 70°-80°C. This makes it possible to laminate the material between two cylinders, at least one of which is heated, using a temperature below that at which the fibers and the film . soften and therefore without melting these components. This makes it possible to obtain a particularly soft and fluffy material. Moreover, since the film is subject to a much lower temperature with respect to that at which it softens, the risk of creating micro-holes in the film itself, which would compromise the impermeability, is avoided.

Ideal adhesives for this purpose are, for example, the heat-sensitive adhesives with codes VH18B and SP507 produced and distributed by Savaré I.C. S.r.l., Milan, Italy.

Generally heat-activated adhesives which have a vitreous transition temperature (Tg) lower than 20°C and preferably lower than 10°C and advantageously with an elastic modulus lower than 10⁷ dynes/cm² are particularly suitable for this purpose. The low vitreous transition temperature makes it possible to obtain particularly soft products.

The film is preferably a thermoplastic film. The thermoplastic material of which the film consists can be selected from a group including: all types of polyolefins, especially polyethylene, polypropylene, amorphous polyolefins and similar products; materials containing meltable components which include polymeric fibers or binders, including natural fibers such as cellulose, wood cellulose, cotton, jute, etc., synthetic fibers such as fibers of glass, rayon®, polyesters, polyolefins, polyamides, acrylic fibers, aromatic amides, polyamides, polytetrafluoroethylene, binders such as high/low melt bicomponent polymer structures, polyester copolymers, polyvinyl chloride, chloride/polyvinyl acetate copolymers, polyamide copolymers, materials formed of mixtures in which one of the components is not meltable; steam or air permeable materials, including microporous film, such as those supplied by the Exxon Chemical Company (USA) under the brand EXXAIRE® or those supplied by Mitsui Toatsu Company (Japan) under the brand ESPOIR NO® and including polymer based monolithic films permeable to steam such as PEBAX® supplied by Elf Atochem (France) or HYTREL® supplied by the Du Pont Company (USA).

The adhesive can be applied using a distributor which applies the adhesive to the film in a random and discontinuous manner. Distributors of this kind are already known. The distribution techniques may be dotting, continuous thread, microthread (for example meltblown or spirobond) or others. Once the adhesive has been distributed discontinuously on the film, localized and discontinuous adhesion is obtained which will offer greater fluffiness, since the fibers adhere to the film in limited areas, while in the areas free of adhesive the fibers will not adhere to the film, but only to one another due only to the effect of reciprocal mechanical grip.

Generally the lamination between the film and web of fibers can be obtained between two smooth cylinders, which can be at room temperature. Alternatively one or both of the two cylinders can be heated. In this case the consolidation occurs between fiber and adhesive. The heating of one or both of the cylinders can be used to activate the adhesive. According to a possible alternative embodiment of the invention, the lamination of the film and the web of textile fibers can be effected between a smooth cylinder and a cylinder covered with protuberances. Heating one or the other of the two cylinders, or both, it is possible to obtain a partial melting of at least some of the fibers of the web only in the areas corresponding to the protuberances on the cylinder and a lower heating in the area between the protuberances- Thus the web of textile fibers is anchored to the plastic film through partial spot bonding in correspondence with the protuberances, while in the areas between contiguous protuberances the temperature will be sufficient to activate the heat-sensitive adhesive, with consequent adhesion of the fibers to the film by gluing.

In this case too, the adhesive is applied in a discontinuous manner as required. The anchorage between the fibers and the plastic film through gluing is less tenacious than anchorage obtained by melting in correspondence with the protuberances on the laminating cylinder, however the web of textile fibers will be soft and fluffy in the areas between the adjacent protuberances.

Alternatively, the temperature of the areas of compression between the smooth cylinder and the protuberances can be lower than the melt temperature, but high enough to activate the adhesive. In this case adhesion occurs exclusively thanks to the effect of the adhesive and exclusively in the areas of the protuberances, thus the product remains soft.

To avoid excessive rigidity of the material and to maintain the desired characteristics of fluffiness, softness and textile feel it is preferable for the surface on which the fibers are partially melted to be equal to a maximum of 25% of the total surface of the film and preferably less than 15%.

To obtain localized anchorage by melting the fibers avoiding damage to the film below, is advantageous if the fibers consist, at least in part, of a polymer with a softening temperature below that of the polymer or polymers of which the film is made. This is possible if fibers with different characteristics are mixed, some with a low melting temperature and others with a high melting temperature. Thus the areas where the protuberances act will cause only partial melting and only of the fibers. Alternatively it is possible to use bicomponent fibers, i.e. fibers with a core with a high melting temperature and a sheath with a lower melting temperature, It is also possible to use a combination of these two solutions. In any case, and whichever solution is chosen (mixture of fibers and/or bicomponent fibers), it is advantageous that a proportion of the fibers have a lower melting temperature than the others.

With the process according to the invention the result is basically a sheet material comprising a plastic film and a web of textile fibers combined to the plastic film, In which the fibers of the web are unconsolidated together at least on a part of the surface area of the sheet material and are united to the film at least in part by means of an adhesive. In practice, as stated above the fibers can be consolidated by means of partial melting in certain points or areas, in which the protuberances of the laminating cylinder have acted, while in the adjacent areas the fibers are free or fixed only to the plastic film by means of a discontinuous application of adhesive.

The film can be previously supplied with through apertures. Or the composite sheet material can be perforated after the combining of the fibers and the film. The apertures are provided when the sheet product is used in those applications in which a fluid must pass from one side of the sheet material to the other, for example when it is used as the upper layer or top sheet of an absorbent product, such as a sanitary towel, a diaper or other item. The back sheet of this type of articles can also be made of sheet material according to the invention. In both cases the user will find the absorbent product very comfortable to wear.

The sheet material can also be used for the creation of other products, in any occasion in which it is necessary to use a plastic sheet material which gives a very comfortable fabric feel. For example the sheet material according to this invention can be used for making tablecloths, examination table sheets, overalls and masks for use in the health services or other uses. Depending on the use of the product the plastic film can be apertured or integral.

Moreover it is possible to apply two webs of fibers to the plastic film, one on each side. In this case at least one of the two webs is applied using the process according to the present invention, but preferably both webs will be applied using this process to obtain a very soft final product. The sheet material with a double web can be created with webs of the same type on both sides, or with webs having different characteristics on each side. In particular, it is possible for one web to have mainly hydrophile characteristics and the other mainly hydrophobic characteristics. In this case the plastic film is preferably apertured and the sheet material is particularly suitable as top sheet of absorbent sanitary articles.

Other advantageous characteristics and embodiments of the process and the product according to the invention are given in the attached claims.

### Brief description of the Drawings

The invention will be better understood by reading the description and examining the enclosed drawing which shows practical but not limiting embodiments of the invention. The drawing, in which the same numbers indicate the same or corresponding parts show:
Fig. 1: a simplified drawing of the plant for the production of the combined sheet material according to the method of the present invention;
Fig. 2: a schematic and greatly enlarged section of the sheet material according to a first embodiment;
Fig. 3: a plan view of Fig. 2 along the line III-III;
Fig. 4: a section similar to that in Fig. 2 of the material according to a different embodiment;
Fig. 5: a plan view of Fig. 4, along the line V-V on a different scale;
Figs. 6a - 6d: schematic sections of the material in successive stages of the work process;
Fig. 7: a plan view similar to the view in Fig. 3, with different distribution of the adhesive;
Fig. 8: a diagram of the plant modified with respect to the plant shown in Fig. 1,
Fig. 9 a section of the sheet material with two webs of fibers on both sides of the film;
Fig. 10 a section of a combined sheet material in which the film is apertured;
Fig. 11 a cross section schematic view of an absorbent product in which sheet material according to the invention is used.

### Detailed Description of Preferred Embodiments of the Invention

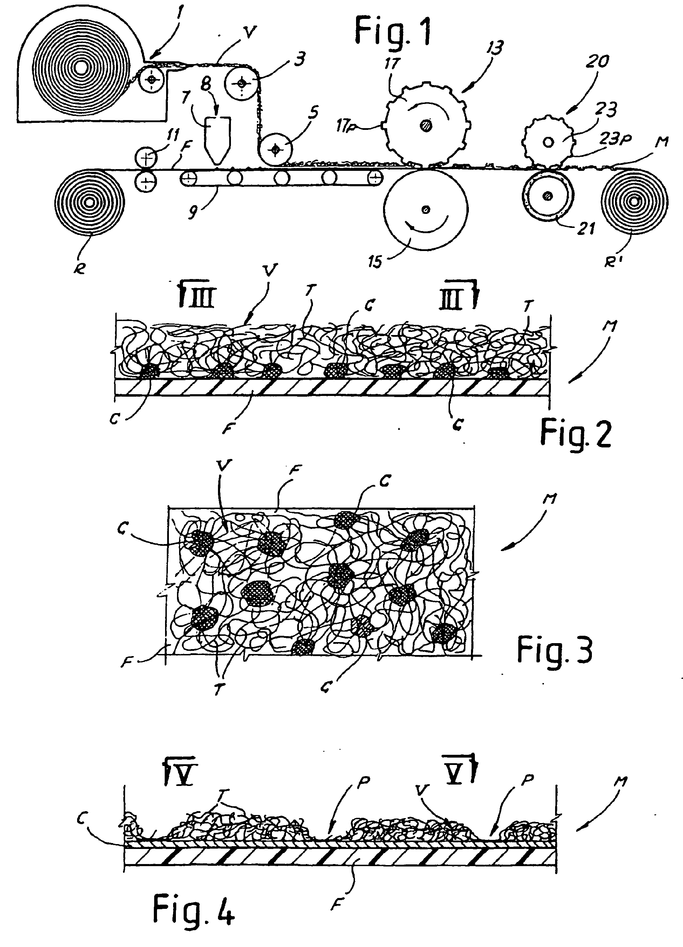

Fig. 1 shows a highly simplified drawing of the most essential parts of a plant for the production of combined sheet material. The plant comprises a machine 1 for the production of a web V of un-consolidated textile fibers. The machine 1 is schematically shown as a carding machine since the carded web is currently the preferred form of web for the production of the material. However the use of different machines or devices for the production of webs of un-consolidated textile fibers for use in the present method is not to be excluded.

The web V of un-consolidated textile fibers is fed, through the first feeders schematically shown as a series of rollers 3, 5 toward a work area. The web will be suitably supported by, for example, a system of conveyor belts - not shown - to overcome the lack of mechanical resistance of the web itself.

The fibers making up the web V may be natural fibers, for example cotton fibers. However, according to the preferred embodiment of the invention the fibers are synthetic, for example polypropylene, polyethylene, nylon®, polyester, rayon®, cellulose acetate or similar, of a length typically between 20 and 60 mm and, for example a count between 1 and 25 dtex. The thickness of the web V of loose fibers is typically between 0.2 and 5 mm, but this is not binding.

A plastic film F, for example a film of polyethylene, of suitable thickness, typically 10 - 50 µm and preferably between 15 and 25 µm fed from a supply bobbin or roll R is placed next to a web V of textile fibers. Before it is placed next to the web V of un-consolidated textile fibers an adhesive is applied to the film F by means of a suitable dispenser 7 positioned on an adhesive station 8. The application may be carried out by spreading, spray or other known system, to obtain an essentially continuous layer, or a discontinuous and random distribution of adhesive on the surface toward the upper side of the film F. The quantity of adhesive distributed on the film is typically between 0.1 and 15 g/m² and preferably between 0.2 and 10 g/m² and even more preferably between 0.5 and 5 g/m². When the distribution is discontinuous and random the amount of adhesive distributed per surface unit should in any case remain substantially uniform.

The adhesive is preferably a heat-sensitive adhesive (hot melt) with an activation temperature advantageously between 70° and 80°C and a vitreous transition temperature (Tg) lower than 20°C and preferably below 10°C to maintain a high level of softness even at room temperature.

Along a conveyor belt 9, which in the example in the drawing forms - together with a pair of feeder rollers (1 - the plastic film feeders F, the web V of un-consolidated textile fibers and the plastic film F are placed next to each other, to be fed into a laminator group 13 consisting in the example illustrated of a smooth lower cylinder 15 and an upper cylinder 17 provided with protuberances or projections 17P. The lower cylinder 15 will preferably have an outer surface in steel or other essentially rigid material, which is similar to the material which forms the outer surface and the protuberances of the upper cylinder 17. The possibility that the lower cylinder may be covered in a yielding material such as rubber, is not to be excluded.

One or both the cylinders 15, 17 can be heated to a temperature sufficient to activate the adhesive C applied by the dispenser, when this is a hot melt adhesive. As will be explained below the lamination temperature can be of a value which will cause the partial melting of the fibers forming the web V. In particular the temperature and the pressure exercised by the lamination group 13 can be such as to cause at least the partial melting of the un-consolidated textile fibers in correspondence with the protuberances 17P of the upper cylinder 17.

In the example illustrated in Fig. 1 an embossing group 20 comprising a first lower embossing cylinder 21 with a smooth surface, preferably in rubber, and a second upper embossing cylinder 23 covered with protuberances 23P is foreseen downstram of the laminator group 13. When required the embossing group 20 embosses the sheet material M obtained by combining through lamination of the plastic film F and web V of textile fibers. The sheet material M is then wound onto rolls R'.

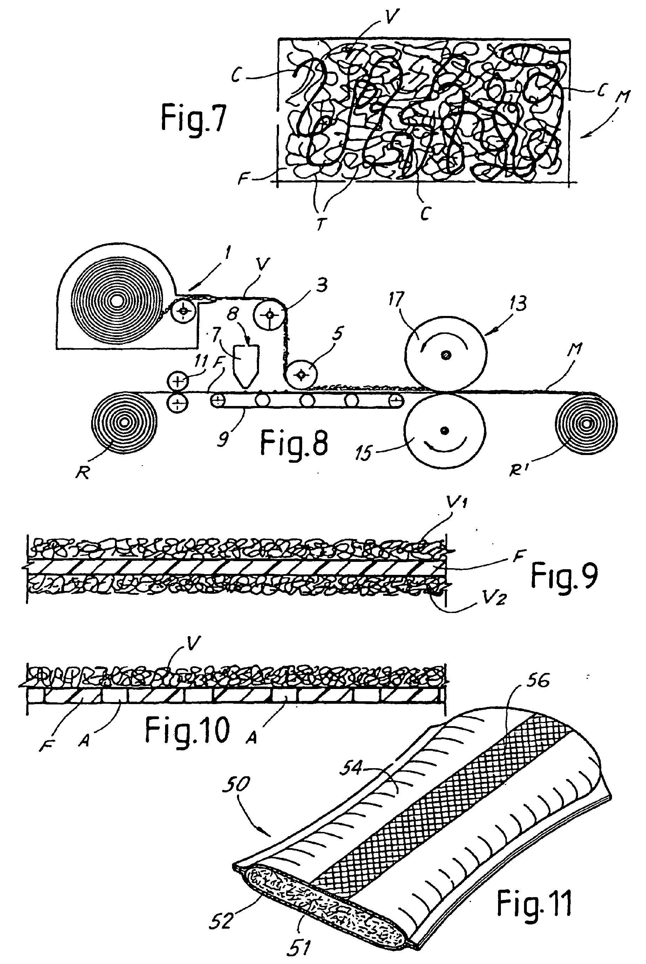

In Fig. 8 a modified plant is shown schematically, in which the upper cylinder 17 is smooth as is the lower cylinder 15 and in which the embossing group 20 is not provided.

Figs. 2 and 3 schematically show the sheet material M obtained from the plant described above when the adhesive C is applied in a random and discontinuous manner and a pair of smooth laminating cylinders 15, 17 is used (plant in Fig. 8). On passing through the laminator group 13 the adhesive C is activated thermally bringing it to the softening temperature, so that the textile fibers T forming the web V adhere to the plastic film F below in areas. The areas in which it adheres are more widely spread according to the distribution of the adhesive C. The density of the distribution of the adhesive depends also on the length of the fibers, since it is advisable that on average each fiber should have at least one point of anchorage to the film F. Therefore the shorter the fibers the denser the dots of adhesive C.

In the areas without adhesive the textile fibers T are joined together only by the mechanical effects of friction, as in the web V of unconsolidated textile fibers. The film F therefore has considerable surface fluffiness and softness. The sheet material M remains flexible also thanks to the discontinuous distribution of the adhesive C.

On the other hand Fgs 4 and 5 show, schematically, the sheet material obtained using even distribution of the adhesive C and a laminator cylinder 17 provided with protuberances 17P (Fig. 1) heated to a temperature sufficient to activate the adhesive in the areas corresponding to the protuberances 17P or to a higher temperature sufficient to cause at least a partial melting of the textile fibers T in the areas of the protuberances 17P on the laminator cylinder 17. The protuberances 17P generate bonding points P (if the fibers melt) or adhesion (if only the adhesive is activated) between the web V of the textile fibers T and the plastic film F. In these areas the textile fibers T are consolidated by the effect of the partial melting or the effect of the adhesive.

When the web V is formed of a mixture of textile fibers T with differing melting temperatures, if the temperature to which the web is heated by the protuberances 17P is between the minimum and the maximum melting temperatures of the fibers in the mixture, some of the textile fibers T will melt locally and others will not, creating points P of cohesion between the fibers and adhesion to the plastic film F with melting of a limited number of fibers. Alternatively, the textile fibers T can be, at least partly, of the bicomponent type, with a sheath having a melting temperature lower than the core. In this case the temperature of the protuberances 17P of the laminator cylinder 17 will be sufficient to cause the melting of only the outer part or sheath of the bicomponent fibers.

In the area surrounding the bonding or adhesion points P the textile fibers T of the web V will adhere at least partially to the plastic film P due to the effect of the adhesive C which is heat-activated, if the temperature in the lamination groove is sufficient to reach the thermal activation of the adhesive even in the areas adjacent to the protuberances 17P.

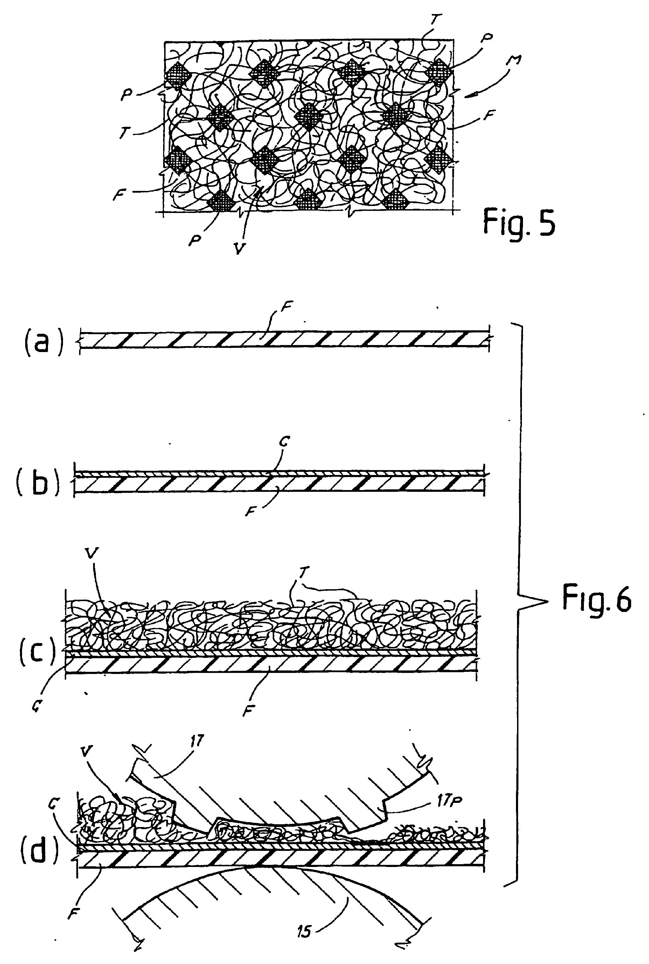

The process of adhesion of the web V of un-consolidated textile fibers T to the plastic film F is shown schematically in Figures 6a - 6d: Fig. 6a shows the plastic film issuing from the roll R. Fig. 6b shows the plastic film F on which the adhesive C has been applied which in this example forms a continuous layer. It should be understood that in this case, too, the distribution of the adhesive could be discontinuous as shown in the example of Figs 2 and 3. In Fig. 6c the web V of un-consolidated textile fibers T has been laid on the plastic film F spread with a layer of adhesive C. Fig. 6d shows the plastic film F and the web V in the lamination nip between the laminator cylinders 15, 17. In the area between the two consecutive protuberances 17P shown in Fig. 6d the temperature (obtained by heating the cylinder 17 and/or the cylinder 15) is sufficient to activate the adhesive C, but not sufficient to soften the textile fibers T and/or the plastic film F. In the points where the protuberances 17P act the textile fibers T are at least partially softened and melted.

The adhesion of the textile fibers T to the plastic film F in the areas around the bonding points P is not as strong as in the adhesion points themselves, since the adhesive makes the fibers adhere to the plastic film F in a more elastic manner. Moreover the thickness of the layer of adhesive C is chosen, with respect to the thickness of the web V of textile fibers T so that only the fibers closest to the plastic film F and therefore to the layer of adhesive C will be enclosed in the adhesive and made to adhere. Thus on the one hand a particularly reliable reciprocal adhesion between the web V and the film F is obtained thanks to the bonding points, and on the other hand the product is soft and fluffy thanks to the areas around the bonding points. The softness is even increased if the adhesive is spread discontinuously.

Alternatively, if the temperature in the lamination nip is lower than the activation temperature of the adhesive, except in correspondence with the protuberances 17P, combining of the fibers will occur only in correspondence with the protuberances 17P, white in the surrounding areas the fibers will remain mainly non consolidated and will not adhere to the film. Alternatively the temperature can be such that it causes the complete activation of the adhesive in correspondence with the protuberances 17P and only partially in the surrounding areas, with the consequent more marked adhesion in the areas of the protuberances and less marked in the adjacent areas.

The protuberances 17P may have a pitch comprised between 0.5 and 15 mm and preferably less than 10 mm and they may have a front surface which increases with the increase in the pitch, for example between 0.2 and 2.5 mm². The density and the dimensions of the protuberances 17P are preferably such as to cause bonding which involves not more than 25% of the total surface of the sheet material M.

Fig. 7 shows a plan view similar to Fig. 3, in which the adhesive C is applied in the form of casual threads, rather than drops or in discrete areas. The method for anchoring the fibers can be as already described. The form taken by the adhesive applied to the film depends on the application technique used.

In Fig. 9 shows an embodiment in which two distinct webs of textile fibers V1 and V2 are applied to the film F, on both sides of the film. The application can be carried out using the method as that described for a single web. For example, the two webs can be placed next to the film F after a layer of adhesive has been applied to both sides. Then the film and the two webs of textile fiber are laminated between two cylinders 15 and 17.

When the composite material is used for the production of a top sheet of an absorbent product, or for other uses in which permeability to liquids is necessary, the plastic film F can have apertures or through holes. These can be created in the film before it is placed next to the web of textile fibers, possibly during a previous work process or in line with the lamination process. For example a perforator group can be mounted between the roll R and the conveyor 9. Alternatively the perforation can be created below the laminator group 13. Fig. 10 shows a schematic cross section of sheet material including an apertured plastic film F with through holes A. The apertures can be made using one of the various known methods which make it possible to create a three-dimensional structure, that is an apertured film whose thickness is greater than the thickness of the integral film before perforating. A suitable technique for obtaining this three-dimensional effect is described, for example, in EP-A-0598970 by the same owner, to which reference can be made for more detail.

Fig. 11 schematically illustrates a sectioned portion of an absorbent product 50 of the type including an absorbent core 51, an impermeable back sheet 52 and a front or top sheet permeable to liquids 54. Reference 56 designates an apertured area of the top sheet. The back sheet and/or the top sheet of the product 50 can be created with a sheet material of the type described above. In particular the top sheet will be formed of a plastic film F provided with apertures (A) as shown schematically in Fig. 10. A double web of textile fibers can be applied to the top sheet as shown in the structure in Fig. 9, with the two webs, respectively internal and external, having different characteristics: hydrophile or hydrophobic.

It should be understood that the drawing show only possible embodiments of the invention, which can vary in the form and arrangement, while remaining within the concept of the invention, as defined in the claims which follow. Any reference numbers in the claims have the sole purpose of facilitating the reading therof with reference to the attached drawing and do not limit the scope of protection in any way.

## Claims

1. A process for the production of a composite sheet material comprising a plastic film (F) and a web (V) of textile fibers en combined with said plastic film (F), including the following steps:
a) feeding the plastic film (F) to a lamination station;
b) feeding the web (V) to said lamination station;
c) discontinuously and randomly applying an adhesive to one surface of the film or to one surface of the web;
d) applying the web (V) to said plastic film (F) with the adhesive distributed between the web and the film:
e) laminating the plastic film and the web causing reciprocal adhesion, between the web of un-consolidated textile fibers and the plastic film by means of said adhesive,
**characterized in that** said web is an unconsolidated carded web of textile fibers and is applied to said film while still unconsolidated.

2. Process according to claim 1, **characterized in that** said fibers have a length between 20 and 60 mm.

3. Process according to claim 1 or 2, **characterized in that** said web has a thickness of between 0.2 and 5 mm.

4. Process according to claim 1, 2 or 3, **characterized in that** said adhesive is applied to the film.

5. Process according to one or more of the previous claims, **characterized by** causing at least a partial consolidation of the web of fibers during lamination.

6. Process according to one or more of the previous claims, **characterized in that** said web of un-consolidated textile fibers is produced in line and directly applied to said plastic film.

7. Process according to one or more of the previous claims, **characterized in that** the web of textile fibers and the film are laminated between two smooth cylinders.

8. Process according to one or more of the previous claims, **characterized in that** said plastic film and said web of un-consolidated textile fibers are heat-laminated, between two cylinders of which at least one is heated.

9. Process according to one or more of the previous claims, **characterized in that** the adhesive is a heat-sensitive adhesive.

10. Process according to claims 8 and 9, **characterized in that** during lamination the plastic film, the adhesive and the web of unconsolidated textile fibers are heated to a temperature sufficient to activate the adhesive at least in certain areas, but lower than the softening temperature of the plastic film and the textile fibers.

11. Process according to claims 8 and 9, **characterized in that** the web of un-consolidated textile fibers is at least partially made up of synthetic fibers and that the plastic film, the adhesive and the web of unconsolidated textile fibers are heated to a temperature sufficient to activate, at least in some areas, the adhesive and cause the softening of at least some of said fibers.

12. Process according to claim 11, **characterized in that** the fibers of said web of un-consolidated textile fibers are consisting of at least two types of fiber with two different softening temperatures.

13. Process according to one or more of the previous claims, **characterized in that** said plastic film and said web of un-consolidated textile fibers are laminated using a laminating cylinder provided with protuberances.

14. Process according to claims 10 and 13 or 11 and 13, **characterized in that** the adhesive is activated thermally in correspondence with the protuberances on said cylinder, to cause dotted adhesion between the web of textile fibers and the plastic film while in the areas between adjacent protuberances the fibers of the web remain essentially non consolidated and do not adhere to the plastic film.

15. Process according to claims 11 and 13, **characterized in that** in correspondence with the protuberances on the laminating cylinder reciprocal adhesion is caused between the plastic film and the web of textile fibers due to at least partial melting of the fibers and/or the film, and that in the areas between said protuberances the web of textile fibers and the plastic film are united by activation of the heat-sensitive adhesive, without melting either the textile fibers and/or the plastic film.

16. Process according to claim 15, **characterized in that** the area in which the fibers are melted and the union between textile fibers and plastic film due to bonding is obtained is equal to or less than 25% of the entire area of the film treated.

17. Process according to one or more of the previous claims, **characterized in that** after lamination the sheet material obtained by the combining of the plastic film and the web of textile fibers is embossed.

18. Process according to one or more of the previous claims, **characterized in that** said film is provided with through apertures.

19. Process according to one or more of the previous claims, **characterized by** the application of a web of fiber to both surfaces of said film.

20. Process according to claim 19, **characterized in that** one of said webs has mainly hydrophile characteristics and the other web has mainly hydrophobic characteristics.

21. Process according to one or more of the previous claims, **characterized in that** said fibers remain non consolidated on at least a portion of the surface of the composite sheet material obtained.

22. Process according to one or more of the previous claims, **characterized by** the use of a heat-activated adhesive with vitreous transition temperature of less than 20°C and preferably less than 10°C.

23. A sheet material comprising a plastic film (F) and a web (V) of textile fibers (T) combined with said plastic film (F), wherein said web is joined to said film by an adhesive (C) which is randomly and discontinuously distributed on at least part of the contact surface between the web and the plastic film, **characterized in that** said web is a carded web of unconsolidated fibers.

24. Sheet material according to claim 23, **characterized in that** said fibers have a length between 20 and 60 mm.

25. Sheet material according to claim 23 or 24, **characterised in that** said web has a thickness between 0.2 and 5 mm.

26. Sheet material according to one or more of claim 23 to 25, **characterized in that** the textile fibers of the web are synthetic fibers consolidated in discrete areas due to at least partial melting of the fibers and/or gluing.

27. Sheet material according to claim 26, **characterized in that** in at least some of said discrete areas in which the textile fibers are consolidated the web is also anchored to the plastic film due also to. bonding together of the fibers and the film.

28. Sheet material according to claim 27, **characterized in that** in the portion adjacent to said discrete areas, in which the fibers are anchored to the plastic film due to bonding, said fibers are joined to the plastic film by gluing.

29. Sheet material according to one or more of claims 23 to 28, **characterized in that** the adhesive which unites said web of textile fibers and said plastic film is a heat-sensitive adhesive.

30. Sheet material according to claim 29, **characterized in that** said adhesive has a vitreous transition temperature of less than 20°C and preferably of less than 10°C.

31. Sheet material according to one or more of claims 23 to 30, **characterized in that** it is at least partially embossed.

32. Sheet material according to one or more of claims 23 to 31, **characterized by** the presence of at least one portion equipped with through apertures.

33. Sheet material according to one or more of claims 23 to 32. **characterized in that** it includes a first web of textile fibers applied on the first surface of said film and a second web of textile fibers applied on the second surface of said film.

34. Sheet material according to claim 33, **characterized in that** said first and second webs of textile fibers have different hydrophile and hydrophobic characteristics.

35. Sheet material according to one or more of the claims 23 to 34, **characterized in that** it comprises an adhesive spread in a quantity between 0.1 and 15 g/m² and preferably between 0.2 and 10 g/m².

36. A sanitary product including a top sheet and a back sheet and an absorbent core between said top sheet and said back sheet, **characterized in that** at least one of said top sheet and said back sheet consists of a sheet material according to one or more of claims 23 to 35.

## Patentansprüche

1. Verfahren zur Herstellung eines Verbundbahnmaterials, das eine Kunststofffolie (F) und eine Faserbahn (V) aus Textilfasern (T) kombiniert mit der Kunststofffolie (F) aufweist, welches Verfahren die folgenden Schritte aufweist:
a) Zuführen der Kunststofffolie (F) zu einer Laminierstation;
b) Zuführen der Faserbahn (V) zu der Laminierstation;
c) Aufbringen eines Klebstoffs diskontinuierlich und wahllos auf eine Oberfläche der Folie oder eine Oberfläche der Faserbahn;
d) Aufbringen der Faserbahn (V) auf die Kunststofffolie (F) mit dem zwischen der Faserbahn und der Folie verteilten Klebstoff:
e) Laminieren der Kunststofffolie und der Faserbahn unter Verursachung gegenseitiger Anhaftung zwischen der Faserbahn aus unverfestigten Fasern und der Kunststofffolie mittels des Klebstoffs,
**dadurch gekennzeichnet, dass** die Faserbahn eine unverfestigte kardierte Faserbahn aus Textilfasern ist und auf die Folie im noch unverfestigten Zustand aufgebracht wird.

2. Verfahren nach Anspruch 1, **dadurch gekennzeichnet, dass** die Fasern eine Länge zwischen 20 und 60 mm aufweisen.

3. Verfahren nach Anspruch 1 oder 2, **dadurch gekennzeichnet, dass** die Faserbahn eine Dicke von zwischen 0,2 und 5 mm aufweist.

4. Verfahren nach Anspruch 1.2 oder 3, **dadurch gekennzeichnet, dass** der Klebstoff auf die Folie aufgebracht wird.

5. Verfahren nach einem oder mehreren der vorhergehenden Ansprüche, **dadurch gekennzeichnet, dass** zumindest eine Teilverfestigung der Faserbahn während der Laminierung verursacht wird.

6. Verfahren nach einem oder mehreren der vorhergehenden Ansprüche, **dadurch gekennzeichnet, dass** die Faserbahn aus unverfestigten Textilfasern mitlaufend hergestellt wird und direkt auf die genannten Kunststofffolie aufgebracht wird.

7. Verfahren nach einem oder mehreren der vorhergehenden Ansprüche, **dadurch gekennzeichnet dass** die Faserbahn aus Textilfasern und die Folie zwischen zwei glatten Zylindern laminiert werden.

8. Verfahren nach einem oder mehreren der vorhergehenden Ansprüche, **dadurch gekennzeichnet, dass** die Kunststofffolie und die Faserbahn aus unverfestigten Textilfasern zwischen zwei Zylindern wärmelaminiert werden, von denen mindestens einer erhitzt ist.

9. Verfahren nach einem oder mehreren der vorhergehenden Ansprüche, **dadurch gekennzeichnet, dass** der Klebstoff ein wärmeempfindlicher Klebstoff ist.

10. Verfahren nach den Ansprüchen 8 und 9, **dadurch gekennzeichnet, dass** während der Laminierung der Kunststofffolie, der Klebstoff und die Faserbahn aus unverfestigten Textilfasern auf eine Temperatur erhitzt werden, die zum Aktivieren des Klebstoffs zumindest in bestimmten Bereichen ausreicht, jedoch niedriger als die Erweichungstemperatur der Kunststofffolie und der Textilfasern ist.

11. Verfahren nach den Ansprüchen 8 und 9, **dadurch gekennzeichnet dass** die Faserbahn aus unverfestigten Textilfasern mindestens teilweise aus Kunstfasern besteht, und dass die Kunststofffolie, der Klebstoff und die Faserbahn aus unverfestigten Textilfasern auf eine Temperatur erhitzt werden, die ausreicht, um den Klebstoff zumindest in einigen Bereichen zu aktivieren und die Erweichung zumindest einiger der Fasern zu verursachen.

12. Verfahren nach Anspruch 11, **dadurch gekennzeichnet, dass** die Fasern der Faserbahn aus unverfestigten Textilfasern aus mindestens zwei Typen mit zwei unterschiedlichen Erweichungstemperaturen bestehen.

13. Verfahren nach einem oder mehreren der vorhergehenden Ansprüche, **dadurch gekennzeichnet, dass** die Kunststofffolie und die Faserbahn aus unverfestigten Textilfasern unter Verwendung eines Laminierzylinders laminiert werden, der mit Vorsprüngen versehen ist.

14. Verfahren nach den Ansprüchen 10 und 13 oder 11 und 13, **dadurch gekennzeichnet, dass** der Klebstoff an den Vorsprüngen auf dem Zylinder wärmeaktiviert wird, um punktuelle Haftung zwischen der Faserbahn aus Textilfasern und der Kunststofffolie zu verursachen, während in den Bereichen zwischen benachbarten Vorsprüngen die Fasern der Faserbahn im wesentlichen unverfestigt bleiben und nicht an der Kunststofffolie anhaften.

15. Verfahren nach den Ansprüchen 11 und 13, **dadurch gekennzeichnet, dass** im Bereich der Vorsprünge auf dem Laminierzylinder die gegenseitige Anhaftung zwischen der Kunststofffolie und der Faserbahn aus Textilfasern aufgrund von zumindest teilweisem Schmelzen der Fasern und/oder der Folie verursacht wird, und dass in den Bereichen zwischen den Vorsprüngen die Faserbahn aus Textilfasern und die Kunststofffolie durch Aktivierung des wärmeempfindlichen Klebstoffs ohne Schmelzen entweder der Textilfasern und/oder der Kunststofffolie vereinigt werden.

16. Verfahren nach Anspruch 15, **dadurch gekennzeichnet, dass** die Fläche, in der die Fasern geschmolzen werden und die Vereinigung zwischen Textilfasern und Kunststofffolie aufgrund von Verklebung erhalten wird, gleich oder weniger als 25% der gesamten Fläche der behandelten Folie ist.

17. Verfahren nach einem oder mehreren der vorhergehenden Ansprüche, **dadurch gekennzeichnet, dass** nach der Laminierung das durch Kombinieren der Kunststofffolie und der Faserbahn aus Textilfasern erhaltene Bahnmaterial geprägt wird.

18. Verfahren nach einem der vorhergehenden Ansprüche, **dadurch gekennzeichnet, dass** die Folie mit Löchern versehen wird.

19. Verfahren nach einem oder mehreren der vorhergehenden Ansprüche, **gekennzeichnet durch** die Aufbringung einer Faserbahn auf beide Oberflächen der Folie.

20. Verfahren nach Anspruch 19, **dadurch gekennzeichnet, dass** eine der Faserbahnen hauptsächlich hydrophile Charakteristiken aufweist und die andere Faserbahn hauptsächlich hydrophobe Charakteristiken hat.

21. Verfahren nach einem oder mehreren der vorhergehenden Ansprüche, **dadurch gekennzeichnet, dass** die Fasern auf mindestens einem Teil der Oberfläche des erhaltenen Verbundbahnmaterials unverfestigt bleiben.

22. Verfahren nach einem oder mehreren der vorhergehenden Ansprüche, **gekennzeichnet durch** die Verwendung eines wärmeaktivierten Klebstoffs mit einer Glasübergangstemperatur von weniger als 20°C und vorzugsweise weniger als 10°C.

23. Bahnmaterial, das eine Kunststofffolie (F) und eine Faserbahn (V) aus Textilfasern (F) kombiniert mit der Kunststofffolie (F) aufweist, wobei die Faserbahn mit der Folie durch einen Klebstoff (C) verbunden wird, der wahllos und diskontinuierlich auf mindestens einem Teil der Kontaktfläche zwischen der Faserbahn und der Kunststofffolie verteilt wird, **dadurch gekennzeichnet, dass** die Faserbahn eine kardierte Faserbahn aus unverfestigten Fasern ist.

24. Bahnmaterial nach Anspruch 23, **dadurch gekennzeichnet, dass** die Fasern eine Länge zwischen 20 und 60 mm aufweisen.

25. Bahnmaterial nach Anspruch 23 oder 24, **dadurch gekennzeichnet, dass** die Faserbahn eine Dicke zwischen 0,2 und 5 mm aufweist.

26. Bahnmaterial nach einem oder mehreren der Ansprüche 23 bis 25, **dadurch gekennzeichnet, dass** die Textilfasern der Faserbahn Kunstfasern sind, die in getrennten Bereichen durch zumindest teilweises Schmelzen der Fasern und/oder Kleben verfestigt werden.

27. Bahnmaterial nach Anspruch 26, **dadurch gekennzeichnet, dass** in mindestens einigen der getrennten Bereiche, in denen die Textilfasern verfestigt sind, die Faserbahn auch an der Kunststofffolie auch durch Verbinden der Fasern und der Folie verankert wird.

28. Bahnmaterial nach Anspruch 27, **dadurch gekennzeichnet, dass** in dem Teil angrenzend an die getrennten Bereiche, in denen die Fasern durch Bindung an der Kunststofffolie verankert sind, die Fasern durch Kleben mit der Kunststofffolie verbunden werden.

29. Bahnmaterial nach einem oder mehreren der Ansprüche 23 bis 28, **dadurch gekennzeichnet, dass** der Klebstoff, der die Faserbahn aus Textilfasern und die Kunststofffolie vereinigt, ein wärmeempfindlicher Klebstoff ist.

30. Bahnmaterial nach Anspruch 28, **dadurch gekennzeichnet, dass** der Klebstoff eine Glasübergangstemperatur von weniger als 20°C und vorzugsweise von weniger als 10°C aufweist.

31. Bahnmaterial nach einem oder mehreren der Ansprüche 23 bis 30. **dadurch gekennzeichnet, dass** es mindestens teilweise geprägt ist.

32. Bahnmaterial nach einem oder mehreren der Ansprüche 23 bis 31, **gekennzeichnet durch** das Vorliegen mindestens eines mit Löchern ausgestatteten Teils.

33. Bahnmaterial nach einem oder mehreren der Ansprüche 23 bis 32, **dadurch gekennzeichnet, dass** es eine erste Faserbahn aus Textilfasern, die auf die erste Oberfläche der Folie aufgebracht wird, und eine zweite Faserbahn aus Textilfasern einschließt, die auf die zweite Oberfläche der Folie aufgebracht wird.

34. Bahnmaterial nach Anspruch 33, **dadurch gekennzeichnet, dass** die erste und zweite Faserbahn aus Textilfasern unterschiedliche hydrophile und hydrophobe Charakteristiken aufweisen.

35. Bahnmaterial nach einem oder mehreren der Ansprüche 23 bis 34, **dadurch gekennzeichnet, dass** es einen Klebestoff aufweist, der in einer Menge zwischen 0.1 und 15 g/m² und vorzugsweise zwischen 0.2 und 10 g/m² verteilt ist.

36. Hygieneprodukt, das eine obere Bahn und eine rückseitige Lage sowie einen absorbierenden Kern zwischen der oberen Lage und der rückseitigen Lage einschließt, **dadurch gekennzeichnet, dass** von der oberen Lage und der rückseitigen Lage mindestens eine aus einem Bahnmaterial gemäß einem oder mehreren der Ansprüche 23 bis 35 besteht.

## Revendications

1. Procédé de production d'un matériau en feuille composite comprenant un film en matériau synthétique (F) et une bande (V) de fibres textiles (T) combinée avec ledit film en matériau synthétique (F), comprenant les étapes suivantes :
a) l'acheminement du film en matériau synthétique (F) à un poste de stratification;
b) l'acheminement de la bande (V) audit poste de stratification;
c) l'application discontinue et aléatoire d'un adhésif à une seule surface du film ou à une seule surface de la bande;
d) l'application de la bande (V) sur ledit film en matériau synthétique (F) avec l'adhésif distribué entre la bande et le film;
e) la stratification du film en matériau synthétique et de la bande pour provoquer une adhérence mutuelle entre la bande de fibres textiles non consolidées et le film en matériau synthétique au moyen dudit adhésif,
**caractérisé en ce que** ladite bande est une bande cardée non consolidée de fibres textiles et est appliquée audit film tandis qu'il n'est pas encore consolidé.

2. Procédé selon la revendication 1, **caractérisé en ce que** lesdites fibres ont une longueur entre 20 et 60 mm.

3. Procédé selon la revendication 1 ou 2, **caractérisé en ce que** ladite bande a une épaisseur entre 0,2 et 5 mm.

4. Procédé selon la revendication 1, 2 ou 3, **caractérisé en ce que** ledit adhésif est appliqué au film.

5. Procédé selon une ou plusieurs des revendications précédentes, **caractérisé en ce qu'**on provoque au moins une consolidation partielle de ladite bande de fibres pendant la stratification.

6. Procédé selon une ou plusieurs des revendications précédentes, **caractérisé en ce que** ladite bande de fibres textiles non consolidées est produite en ligne et directement appliquée audit film en matériau synthétique.

7. Procédé selon une ou plusieurs des revendications précédentes, **caractérisé en ce que** la bande de fibres textiles et le film sont contrecollés entre deux cylindres lisses.

8. Procédé selon une ou plusieurs des revendications précédentes, **caractérisé en ce que** ledit film en matériau synthétique et ladite bande de fibres textiles non consolidées sont contrecollés à la chaleur entre deux cylindres, dont l'un au moins est chauffé.

9. Procédé selon une ou plusieurs des revendications précédentes, **caractérisé en ce que** l'adhésif est un adhésif sensible à la chaleur.

10. Procédé selon les revendications 8 et 9, **caractérisé en ce que**, au cours de la stratification, le film en matériau synthétique, l'adhésif et la bande de fibres textiles non consolidées sont chauffés à une température suffisante pour activer l'adhésif au moins dans certaines zones, mais inférieure à la température de ramollissement du film en matériau synthétique et des fibres textiles.

11. Procédé selon les revendications 8 et 9, **caractérisé en ce que** la bande de fibres textiles non consolidées est au moins partiellement faite de fibres synthétiques et **en ce que** le film en matériau synthétique, l'adhésif et la bande de fibres textiles non consolidées sont chauffés à une température suffisante pour activer l'adhésif au moins dans certaines zones et provoquer le ramollissement d'au moins certaines desdites fibres.

12. Procédé selon la revendication 11, **caractérisé en ce que** les fibres de ladite bande de fibres textiles non consolidées sont constituées d'au moins deux types de fibre avec deux températures différentes de ramollissement.

13. Procédé selon une ou plusieurs des revendications précédentes, **caractérisé en ce que** ledit film en matériau synthétique et ladite bande de fibres textiles non consolidées sont contrecollés en utilisant un cylindre de stratification muni de protubérances.

14. Procédé selon les revendications 10 et 13 ou 11 et 13, **caractérisé en ce que** l'adhésif est activé thermiquement en correspondance avec les protubérances sur ledit cylindre pour entraîner une adhérence par points entre la bande de fibres textiles et le film en matériau synthétique, tandis que, dans les zones entre des protubérances adjacentes, les fibres de la bande restent essentiellement non consolidées et n'adhèrent pas au film en matériau synthétique.

15. Procédé selon les revendications 11 et 13, **caractérisé en ce qu'**en correspondance avec les protubérances sur le cylindre de stratification, une adhérence réciproque est produite entre le film en matériau synthétique et la bande de fibres textiles, due à une fusion au moins partielle des fibres et/ou du film et **en ce que**, dans les zones entre lesdites protubérances, la bande de fibres textiles et le film en matériau synthétique sont unis par activation de l'adhésif sensible à la chaleur sans fusion des fibres textiles ni/ou du film en matériau synthétique.

16. Procédé selon la revendication 15, **caractérisé en ce que** la surface dans laquelle les fibres sont fondues et la jonction que l'on obtient entre les fibres textiles et le film en matériau synthétique en raison du collage est égale ou inférieure à 25% de la surface entière du film traité.

17. Procédé selon une ou plusieurs des revendications précédentes, **caractérisé en ce qu'**après stratification, le matériau en feuille obtenu par combinaison du film en matériau synthétique et de la bande de fibres textiles est gaufré.

18. Procédé selon une ou plusieurs des revendications précédentes, **caractérisé en ce que** ledit film est pourvu d'ouvertures traversantes.

19. Procédé selon une ou plusieurs des revendications précédentes, **caractérisé par** l'application d'une bande de fibres sur les deux surfaces dudit film.

20. Procédé selon la revendication 19, **caractérisé en ce que** l'une desdites bandes a principalement des caractéristiques hydrophiles et l'autre bande a principalement des caractéristiques hydrophobes.

21. Procédé selon une ou plusieurs des revendications précédentes, **caractérisé en ce que** lesdites fibres demeurent non consolidées sur au moins une partie de la surface du matériau en feuille composite obtenu.

22. Procédé selon une ou plusieurs des revendications précédentes, **caractérisé par** l'utilisation d'un adhésif activé à la chaleur avec une température de transition vitreuse de moins de 20 °C et de préférence de moins de 10 °C.

23. Matériau en feuille comprenant un film en matériau synthétique (F) et une bande (V) de fibres textiles (T) combinée avec ledit film en matériau synthétique (F), dans lequel ladite bande est jointe audit film par un adhésif (C) qui est distribué de façon aléatoire et discontinue sur au moins une partie de la surface de contact entre la bande et le film en matériau synthétique, **caractérisé en ce que** ladite bande est une bande cardée de fibres non consolidées.

24. Procédé selon la revendication 23, **caractérisé en ce que** lesdites fibres ont une longueur entre 20 et 60 mm.

25. Matériau en feuille selon la revendication 23 ou 24, **caractérisé en ce que** ladite bande a une épaisseur entre 0,2 et 5 mm.

26. Matériau en feuille selon une ou plusieurs des revendications 23 à 25, **caractérisé en ce que** les fibres textiles de la bande sont des fibres synthétiques consolidées dans des zones discrètes dues au moins à une fusion au moins partielle des fibres et/ou à un collage.

27. Matériau en feuille selon la revendication 26, **caractérisé en ce que**, dans au moins certaines desdites zones discrètes dans lesquelles les fibres textiles sont consolidées, la bande est également ancrée au film en matériau synthétique en raison également du collage mutuel des fibres et du film.

28. Matériau en feuille selon la revendication 27, **caractérisé en ce que**, dans la partie adjacente auxdites zones discrètes dans lesquelles les fibres sont ancrées sur le film en matériau synthétique en raison du collage, lesdites fibres sont jointes au film en matériau synthétique par collage.

29. Matériau en feuille selon une ou plusieurs des revendications 23 à 28, **caractérisé en ce que** l'adhésif qui unit ladite bande de fibres textiles et ledit film en matériau synthétique est un adhésif thermosensible.

30. Matériau en feuille selon la revendication 29, **caractérisé en ce que** ledit adhésif a une température de transition vitreuse de moins de 20°C et, de préférence, de moins de 10°C.

31. Matériau en feuille selon l'une ou plusieurs des revendications 23 à 30, **caractérisé en ce qu'**il est au moins partiellement gaufré.

32. Matériau en feuille selon l'une ou plusieurs des revendications 23 à 31, **caractérisé par** la présence d'au moins une partie équipée d'ouvertures traversantes.

33. Matériau en feuille selon une ou plusieurs des revendications 23 à 32, **caractérisé en ce qu'**il comprend une première bande de fibres textiles appliquée sur la première surface dudit film et une seconde bande de fibres textiles appliquée sur la seconde surface dudit film.

34. Matériau en feuille selon la revendication 33, **caractérisé en ce que** lesdites première et seconde bandes de fibres textiles ont des caractéristiques hydrophiles et hydrophobes différentes.

35. Matériau en feuille selon une ou plusieurs des revendications 23 à 34, **caractérisé en ce qu'**il comprend une masse adhésive étalée en une quantité comprise entre 0,1 et 15 g/m² et, de préférence, entre 0,2 et 10 g/m².

36. Produit sanitaire comprenant une feuille supérieure et une feuille postérieure et un noyau absorbant entre ladite feuille supérieure et ladite feuille postérieure, **caractérisé en ce qu'**au moins l'une desdites feuille supérieure et feuille postérieure est constituée d'un matériau en feuille selon une ou plusieurs des revendications 23 à 35.
